# EUROPEAN PATENT APPLICATION

(11) **EP 3 692 955 A1**
(43) Date of publication of application: **12.08.2020**
(21) Application number: 17929333.7
(22) Date of filing: 08.11.2017
(51) Int. Cl.: A61F 5/11, A61M 35/00

(54) **FINGERNAIL AND TOENAIL CORRECTION DEVICE AND FINGERNAIL AND TOENAIL CORRECTION METHOD**

(30) Priority: 17.10.2017 KR 20170134393
(71) Applicant: International Beauty and Foot-Health Promotion Agency Co., LLC, Bupyeong-gu Incheon 21404 (KR)
(72) Inventor: OH, In Suk, Incheon 21389 (KR)
(74) Representative: Isarpatent
(86) International application number: PCT/KR2017/012608
(87) International publication number: WO 2019/078400

(57) **Abstract**

A fingernail and toenail correction device includes: first and second handle parts, each of which has an insertion part, into which a user's finger is inserted, formed at one end, and which are rotatably connected to each other by being cross-coupled through a hinge member; a body part extending from the other end of the first handle part and arranged in the width direction of a toenail or fingernail; hook parts, the upper ends of which are connected to the body part and the lower ends of which are bent upward so as to be inserted and hooked to both sides of the toenail or fingernail; and a pressing part extending from the other end of the second handle part and contacting the top surface of the toenail or fingernail to press the top surface of the toenail or fingernail.

## Description

### [Technical Field]

The present invention relates to a technique of correcting a fingernail or a toenail, and more particularly, to a nail correction device and a fingernail or a toenail correction method, which allows an ingrown nail digging into the flesh to be smoothly corrected without pain, allows a correction effect to be maintained for a long time, and allows even a beginner to easily correct a nail without additional know-how.

### [Background Art]

An ingrown nail, that is one of nail diseases, is a disease in which a nail grows into the flesh such that inflammation and pain occur. For example, when an edge of a nail is deeply cut by a nail clipper, an uncut piece of the nail hidden in the flesh may grow into the flesh. Otherwise, a case may occur when a toenail is distorted or when tightened shoes are put on for a long time.

As the most major symptom, there are heat, pain, and swelling of an occurrence part. The ingrown nail most generally occurs in a big toe among five toes, and more particularly, in a big toe of a right foot. First, an outside or inside of a big toe turns slightly red and swells with slight pain. Due to continuous friction, the big toe further swells, a sore oozes, granulation tissue (a mass formed due to increase of inflammation, blood vessels, and fibrous tissue) increases, a part around the toenail starts festering, and pain becomes serious.

When the development as described above is left without treatment, due to pain, a disorder may be caused in daily life, for example, in walking or exercising. Also, the inflammation becomes severe, a secondary infection such as cellulitis (a disease in which germs invade subcutaneous tissue and cause a purulent inflammation) may occur. Accordingly, it is necessary to quickly treat the disease. Here, as a treatment method, there are an operation and correction. In a mild case, when a wad of cotton or a dental floss is inserted between an edge of an ingrown nail and the flesh therebelow, an operation is not necessary. However, in a severe case, an operation is necessary. In the operation, side surfaces of an ingrown nail plate are removed after local anesthesia, folds of skin on an edge of a nail, which cover the side surfaces, are removed, and the ingrown nail plate is excised lengthwise in order to prevent recurrence. Here, an outer surface of the nail is also excised or galvanocautery is performed using a high frequency.

In addition to the above treatment methods, naturally treating an ingrown nail using a correction device is well known.

Conventional nail correction devices include an ingrown toenail correction mechanism disclosed in Korean Patent Registration No. 1659219 and a rolled-in nail correction device disclosed in Japanese Patent Registration No. 5684437.

In more detail, these conventional nail correction devices will be described below.

Korean Patent Registration No. 1659219 discloses an ingrown nail correction mechanism including a toenail correction body formed of an elastic material having a certain length, having both ends bent to be opposite to each other, and including holding bent portions formed at the opposite ends to be inserted into both sides of an ingrown toenail to hold the both sides so as to be implanted in a standing manner while providing an elastic force to the ingrown toe nail; and an elasticity adjustment tool including a fixing nut coupled to be reciprocally movable from a certain position of the toenail correction body and located to come into contact with the ingrown toenail to allow practitioners to arbitrarily adjust elastic intensity applied from the toenail correction body to the ingrown toenail according to a degree of a toenail disease, detachably coupled to a certain position of the toenail correction body to provide a guiding screw hole, and including two split nuts separably screw-coupled with a horizontal portion of the toenail correction body therebetween, an adjustment shaft coupled to the fixing nut to be reciprocally movable and including a handle at a top and a pressurizing head portion formed at a bottom, which is located to face the holding bent portion, to come into contact with the toenail, and a spring coupled to the adjustment shaft while being supported between the fixing nut and the pressurizing head portion to provide an elastic pressure to the adjustment shaft.

Japanese Patent Registration No. 5684437 discloses a rolled-in nail correction device including a base body, a left arm including a holding portion on a left side connection portion of a nail at a free end, a right arm including a holding portion on a right side connection portion of the nail at a free end, and a branch member between the left arm and the right arm and having a bottom end as a contact portion for the nail. Here, the left arm and the right arm are combined such that top ends thereof are pivotable on the base body. The branch member includes a shaft body combined to be reciprocated vertically into the base body, an operation body combined with the shaft body to be vertically movable to dispose the corresponding contact portion below the shaft body, and a transient member interposed between the operation body and the shaft body for the operation body. The corresponding contact portion is lengthwise laterally and includes protruding portions on a left end and a right end so as to come into contact with the nail using the protruding portions without contact with the nail between left and right protruding portions.

### [Disclosure]

### [Technical Problem]

The present invention is directed to providing a nail correction device and a nail correction method which allow a nail to be simply and easily corrected at home.

The present invention is directed to providing a nail correction device and a nail correction method, which allow pain to be minimized while a nail is corrected and allow a corrected state to be maintained until the nail is restored a normal state after correction.

The present invention is directed to providing a nail correction device and a nail correction method which allow inconvenience of recorrection necessary for the corrected nail which digs into the flesh again to be prevented.

### [Technical Solution]

One aspect of the present invention provides a nail correction device including a first handle portion and a second handle portion which each have one end at which an insertion portion into which a user's finger is inserted is formed and which are cross-coupled with each other using a hinge member to be pivotably connected to each other, a body portion formed to extend from the other end of the first handle portion and disposed in a lateral direction of a nail, holding portions having top ends connected to the body portion and bottom ends bent upward to penetrate under and hold both ends of the nail, and a pressurizing portion formed to extend from the other end of the second handle portion, to come into contact with a top surface of the nail, and to pressurize the top surface of the nail.

The body portion may include a frame formed in the lateral direction of the nail, first and second arms connected to both ends of the frame, and a connection member configured to connect the frame to the first and second arms such that the first or second arm moves in the lateral direction according to a width of the nail.

The body portion may further include a fixing member configured to fix positions of the first and second arms with an adjusted lateral interval.

The fixing member may be formed as a bolt, and the first or second arm may be formed as a nut.

The holding portions may have a width which gradually decreases in a downward direction.

The holding portions may each include a tapered portion inclined inward in the lateral direction between the top end and the bottom end.

The pressurizing portion may be pivotably connected to the other end of the second handle portion by a hinge.

The first handle portion and the second handle portion may be formed to be perpendicular or parallel to the body portion.

One aspect of the present invention provides a method of correcting a nail. The method includes preparing the nail correction device according to the present invention, applying a solid formulation to a top surface of a nail, disposing the nail correction device on the nail and mounting the holding portions on both ends of the nail, lifting and correcting the both ends of the nail under which the holding portions penetrate while the first handle portion and the second handle portion are gripped and the pressurizing portion comes into contact with the top surface of the nail, maintaining a corrected state of the both ends of the nail for a certain time, and detaching the nail correction device from the nail.

### [Advantageous Effects]

According to one embodiment of the present invention, a nail correction device and a nail correction method can simply and easily correct a nail at home.

According to one embodiment of the present invention, a nail correction device and a nail correction method can minimize pain while a nail is corrected and maintain a corrected state until the corrected nail restores to a normal state after correction.

The nail correction device and the nail correction method according to the present invention can prevent inconvenience of recorrection necessary for the corrected nail which digs into the flesh again.

### [Description of Drawings]

FIG. 1 is a view of a nail correction device according to one embodiment of the present invention.
FIG. 2 is an enlarged view illustrating part A of FIG. 1.
FIG. 3 is a front view of the nail correction device according to one embodiment of the present invention.
FIG. 4 is a view illustrating a fixing member of the nail correction device according to one embodiment of the present invention.
FIG. 5 is a flowchart illustrating a process of correcting a nail using the nail correction device according to one embodiment of the present invention.
FIGS. 6A and 6B are views illustrating a state in which the nail correction device according to one embodiment of the present invention is mounted.
FIG. 7 is a view illustrating a modified example of the state in which the nail correction device according to one embodiment of the present invention is mounted.
FIG. 8 is a photo illustrating a first example in which correction is performed using the nail correction device according to one embodiment of the present invention.
FIG. 9 is a photo illustrating a second example in which correction is performed using the nail correction device according to one embodiment of the present invention.
FIG. 10 is a photo illustrating a third example in which correction is performed using the nail correction device according to one embodiment of the present invention.
FIG. 11 is a photo illustrating a fourth example in which correction is performed using the nail correction device according to one embodiment of the present invention.

### [Modes of the Invention]

Since a description of the present invention corresponds to embodiments for a structural or functional description thereof, the scope of the present invention should not be construed as being restricted by the embodiments described below. That is, since the embodiments may have a variety of changes and a variety of shapes, it should be construed that the scope of the present invention includes equivalents capable of implementing the technical concept thereof. Also, since it is not meant that a particular embodiment includes all the aspects or effects provided by the present invention, the scope of the present invention should not be construed as being restricted thereby.

Meanwhile, the meaning of the terms described herein should be understood as follows.

The term "first," "second," or the like is intended to distinguish one component from another component, and the scope of the present invention should not be limited to the terms. For example, a first component may be referred to as a second component, and similarly, the second component may be referred to as the first component.

When it is stated that one component is "connected" to another component, it should be understood that the one component may be directly connected to the other component but another component may be present therebetween. On the other hand, when it is stated that one component is "directly connected" to another component, it should be understood that no other component is present therebetween. Meanwhile, other expressions for describing a relationship between components, that is, "between," "directly between," "adjacent to," "directly adjacent to," and the like should also be equally construed.

It should be understood that singular expressions, unless clearly defined otherwise in the text, include plural expressions. Also, it should be understood that the terms "comprise," "have," or the like are used herein to specify the presence of stated features, numbers, stages, operations, elements, components or combinations thereof but do not preclude the presence or addition of one or more other features, numbers, stages, operations, elements, components, or combinations thereof.

An identification code (for example, a, b, c, and the like) in each component is used for convenience of description and does not describe an order of components. Also, each component may be configured unlike a specified order unless a particular order is clearly defined in the text.

All the terms used herein, unless defined otherwise, have the same meanings generally understood by one of ordinary skill in the art. Generally, terms defined in generally used dictionaries should be construed as having the meanings which coincide with contextual meanings in the art and cannot be construed as having ideal or excessively formal meanings unless clearly defined herein.

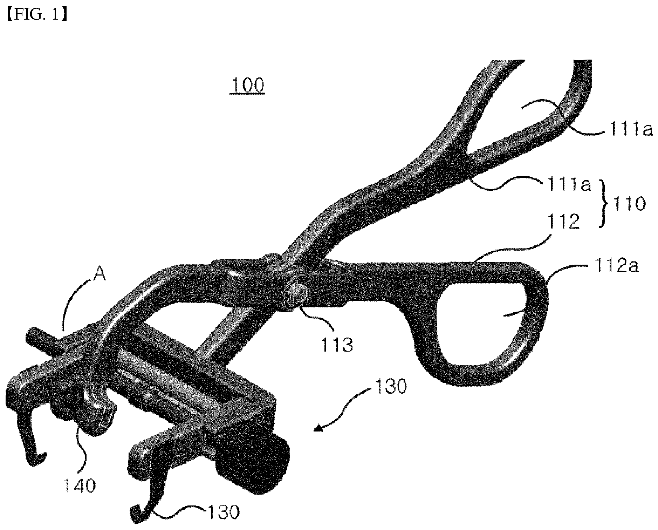

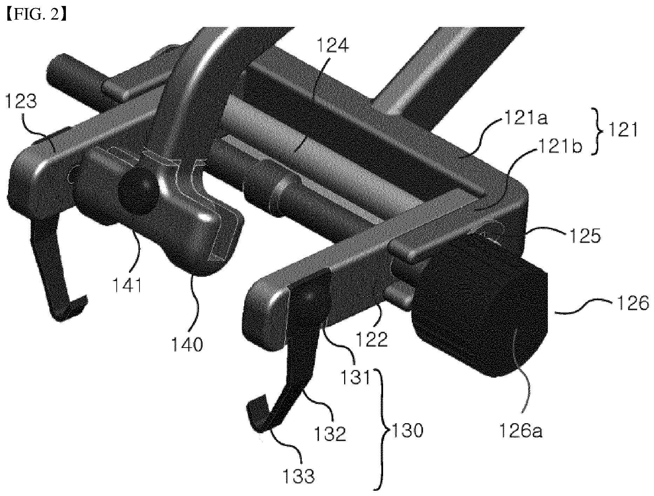

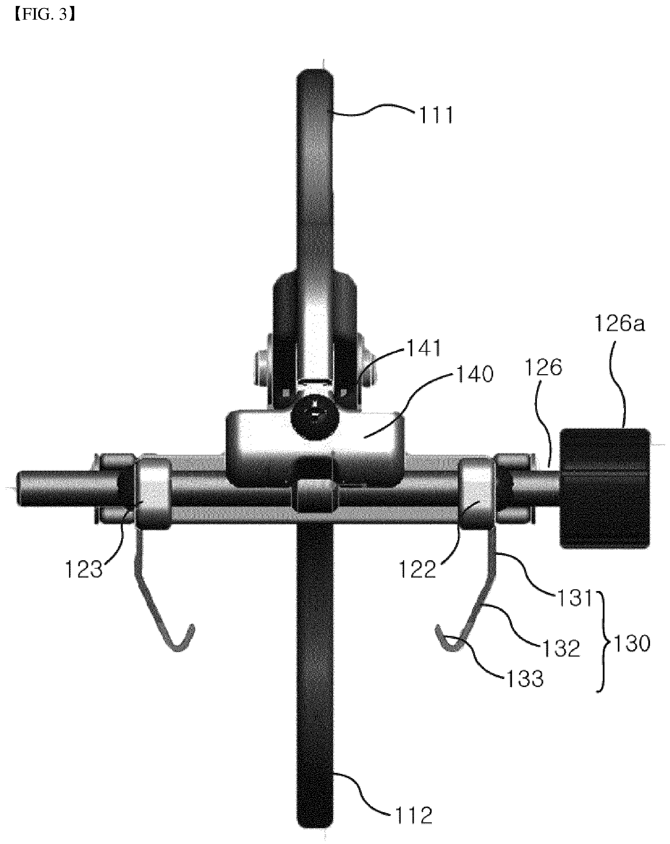

FIG. 1 is a view of a nail correction device according to one embodiment of the present invention, FIG. 2 is an enlarged view illustrating part A of FIG. 1, and FIG. 3 is a front view of the nail correction device according to one embodiment of the present invention.

Referring to FIG. 1, a nail correction device 100 includes a handle portion 110, a body portion 120, holding portions 130, and a pressurizing portion 140.

The handle portion 110 includes a first handle portion 111 and a second handle portion 112 cross-coupled and pivotably connected to each other through a hinge member 113. The first and second handle portions 111 and 112 include insertion portions 111a and 112a, into which user's fingers are inserted, at one ends thereof. The handle portion 110 has a shape similar to scissors. A user inserts fingers into the insertion portions 111a and 112a and increases or decreases an interval between the first and second handle portions 111 and 112 so as to increase or decrease an interval between the other ends of the first and second handle portions 111 and 112 on the basis of the hinge member 113.

The handle portion 110, as shown in FIG. 1, may be formed to be perpendicular to a plane of the body portion 120, but the present invention is not limited thereto and the handle portion 110 may be variously changed in design according to user's convenience. For example, the handle portion 110 may be formed to be parallel to the plane of the body portion 120 or be at a certain angle therewith.

Referring to FIG. 2, the body portion 120 is formed to extend from the other end of the first handle portion 111 and is disposed in a lateral direction of a nail. The body portion 120 includes a frame 121, first and second arms 122 and 123 connected to both sides of the frame 121, and a connection member 124 configured to connect the first and second arms 122 and 123.

The frame 121 includes an extension portion 121a extending in the lateral direction of the nail and protruding portions 121b protruding from both ends of the extension portion 121a in the longitudinal direction of the nail. The first and second arms 122 and 123 are connected to the protruding portions 121b through the connection member 124. The connection member 124 is a bar-shaped member formed lengthwise in the lateral direction of the nail and passes through the protruding portions 121b and the first and second arms 122 and 123 in the lateral direction while the first and second arms 122 and 123 are disposed inside the protruding portions 121b. The connection member 124 is fixed by fastening holes 125 at both ends located outside the protruding portions 121b. In the embodiment, configurations of the connection member are not limited thereto and fastening components having a variety of shapes may be used.

As described above, since the connection member 124 passes through the first and second arms 122 and 123 in the lateral direction, the first and second arms 122 and 123 are movable in the lateral direction of the nail and capable of adjusting an interval between the first and second arms 122 and 123 according to a variety of widths of nails. Here, the interval may be adjusted by moving both the first and second arms 122 and 123 but may be adjusted by moving only one of them.

The body portion 120 may further include a fixing member 126 to fix the interval adjusted by a lateral movement of the first and second arms 122 and 123. The fixing member 126 is disposed to be parallel to the connection member 124 and passes through the protruding portions 121b and the first and second arms 122 and 123 in the lateral direction.

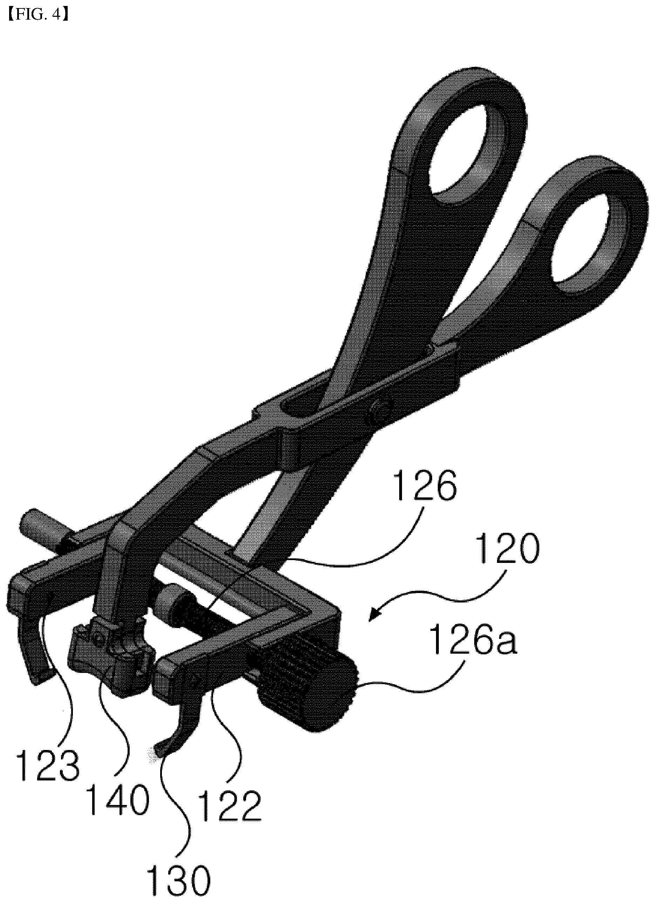

FIG. 4 is a view illustrating the fixing member of the nail correction device according to one embodiment of the present invention. Referring to FIG. 4, the fixing member 126 and the first or second arm 122 or 123 may be screw-coupled. That is, the fixing member 126 is formed as a bolt and the first or second arm 122 or 123 is formed as a nut. A lateral interval is adjusted by moving the first or second arm 122 or 123 in a lateral direction by rotating a head 126a of the fixing member 126. When the lateral interval is adjusted as much as a width of a nail, the head 126a is not further rotated such that screw coupling between the fixing member 126 and the first or second arm 122 or 123 is maintained and positions of the first arms 122 and 123 at the adjusted lateral interval are fixed. Here, it is unnecessary to form both the first and second arms 122 and 123 as nuts, and only the arm moving to adjust the interval may be formed as a nut.

The holding portions 130 include top ends 131 connected to the body portion and bottom ends 133 bent upward to penetrate under and hold both ends of the nail. The top ends 131 are connected to the first and second arms 122 and 123. The bottom ends 133 may be formed to have a hook shape so as to prevent both ends of the nail, which dig into the flesh, from dropping when the both ends of the nail are lifted.

The holding portions 130 may be formed such that widths thereof decrease in a downward direction so as to easily penetrate between the nail and the flesh when the both ends of the nail, which dig into the flesh, are penetrated under and held.

The holding portions 130 may be formed of elastic members so as to be easily operated when penetrating under and holding the both ends of the nail or being separated from the both ends of the nail after correction.

The holding portion 130 may include a tapered portion 132 inclined inward laterally between the top end 131 and the bottom end 133. In consideration of playing a leading role in lifting up the both ends which dig into the flesh while the nail is corrected, the holding portions 130 may be formed of a stiff material and treated to be tapered so as to provide elasticity to the stiff material.

The pressurizing portion 140 is formed to extend from the other end of the second handle portion 112, comes into contact with a top surface of the nail, and pressurizes the top surface of the nail. That is, when the holding portions 130 penetrate under and hold the both ends of the nail, a gap of the handle portion 110 is gradually decreased and the pressurizing portion 140 comes into contact with the top surface of the nail. Here, when the gap of the handle portion 110 is continuously decreased, the both ends of the nail are lifted by a force thereof.

The pressurizing portion 140 may be pivotably connected to the other end of the second handle portion 112 by a hinge 141. An optimal contact state with the top surface of the nail may be maintained by pivoting the pressurizing portion 140 on the hinge 141 according to a shape of the top surface of the nail or a disposition state of the pressurizing portion 140. The present invention is not limited thereto, and the pressurizing portion 140 may be integrated with the second handle portion 112.

The pressurizing portion 140 may include a pad (not shown) on a part which comes into contact with the top surface of the nail. The pad may be formed of rubber or silicone, protect the top surface of the nail which comes into contact with the pressurizing portion 140, and ensure contact with the top surface of the nail.

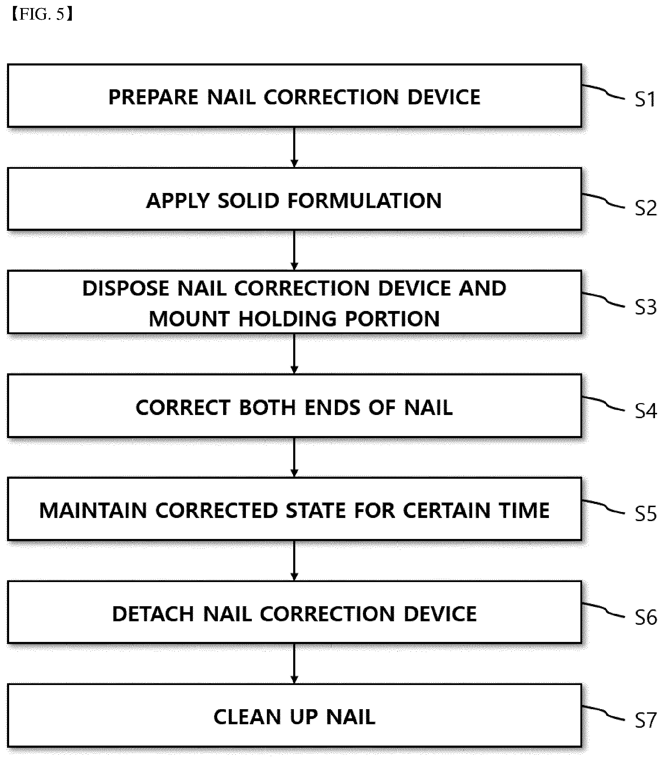

Hereinafter, a correction method using the nail correction device according to one embodiment of the present invention will be described with reference to FIGS. 5 to 7.

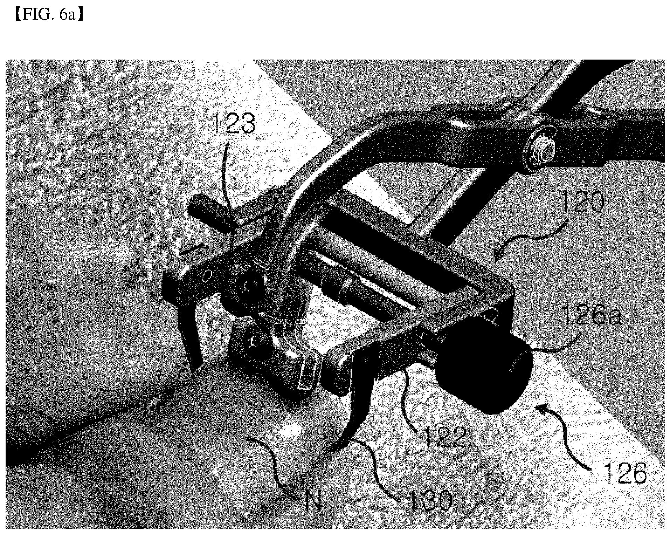

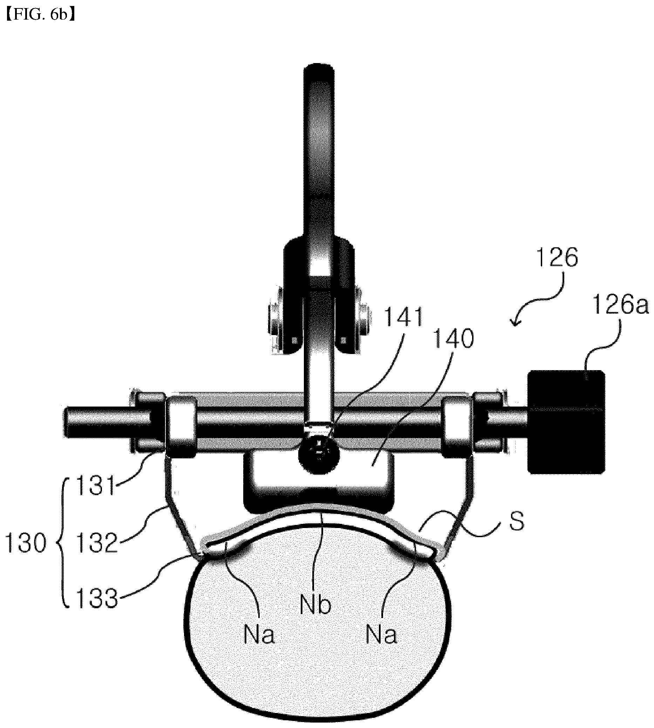

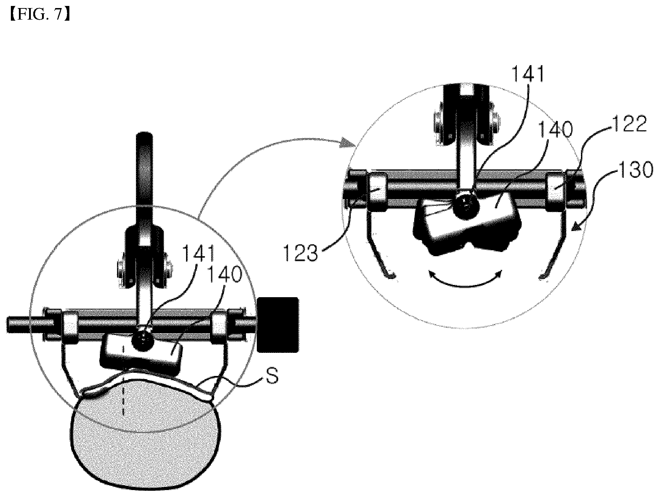

FIG. 5 is a flowchart illustrating a process of correcting a nail using the nail correction device according to one embodiment of the present invention, FIGS. 6A and 6B are views illustrating a state in which the nail correction device according to one embodiment of the present invention is mounted, and FIG. 7 is a view illustrating a state in which a toenail is lifted using the nail correction device according to one embodiment of the present invention.

Referring to FIG. 5, the nail correction method using the nail correction device according to one embodiment of the present invention includes preparing the nail correction device (S1), applying a solid formulation (S2), mounting the nail correction device (S3), correcting both ends of a nail (S4), maintaining a corrected state (S5), detaching the nail correction device (S6), and cleaning up the nail (S7).

The nail correction device is prepared in operation S1, and the solid formulation is applied to a top surface of the nail to be corrected in operation S2. Here, the solid formulation is applied to continuously maintain a state of the corrected nail after correction and may be formed by mixing an acrylic resin liquid with acrylic resin powder. Here, a brush is dipped into the acrylic resin liquid and coated with the acrylic resin powder to apply the acrylic resin liquid and the acrylic resin powder to the top surface of the nail such that the acrylic resin liquid and the acrylic resin powder may be mixed on the top surface of the nail using the brush. Since the nail is corrected according to the embodiment as described above and then a state of the corrected nail is maintained until the nail and the flesh restore a normal state, a situation, in which the nail which digs into the flesh after correction and needs to be recorrected, does not occur.

Meanwhile, in operation S2, the top surface of the nail is sanded before the solid formulation is applied and a thickness of the nail is decreased such that the nail, which was hard, may become thinner and be straightened well. Accordingly, it becomes easy to penetrate the holding portions under both ends of the nail to hold the both ends when the nail correction device is mounted in operation S3.

In operation S3, the nail correction device is disposed on the nail and the holding portions penetrate under and hold both ends of the nail. The body portion 120 is disposed above a nail N and the first or second arm 122 or 123 is moved to be adjusted to a width of the nail N while the head 126a of the fixing member 126 is pivoted. Subsequently, while the bottom ends 133 of the holding portions 130 come into contact with a surface of the nail, the tapered portions 132 of the holding portions 130 are pushed outward such that the bottom ends 133 penetrate into a boundary between the nail and the flesh. Here, upwardly bent parts of the bottom ends 133 are inserted under and hold both ends Na of the nail. FIGS. 6A and 6B illustrate the above-described mounted state. In FIG. 6B, a solid formulation S is applied to the nail.

In operation S4, the first and second handle portions 111 and 112 are gripped and the both ends of the nail are lifted. When the first and second handle portions 111 and 112 are gripped while the pressurizing portion 140 comes into contact with a top surface Nb of the nail, as the first and second handle portions 111 and 112 come close to each other, the pressurizing portion 140 pushes the top surface Nb of the nail due to a force thereof and the holding portions 130 move upward such that the both ends Na of the nail under which the bottom ends 133 of the holding portions 130 are penetrated are held and lifted upward.

Referring to FIG. 7, since the pressurizing portion 140 pivots on the hinge 141, the pressurizing portion 140 is pivoted according to a state of the nail N such that the pressurizing portion 140 may come into contact with the top surface of the nail well.

Subsequently, in operation S5, a state in which the both ends Na of the nail are lifted is maintained for a certain time. Here, the certain time is a time in which the lifted state of the both ends Na of the nail is fixed by the solid formulation and may be less than one minute.

In operation S6, the nail correction device is detached from the nail. In operation S7, the nail is trimmed and cleaned up.

As described above, according to the correction method using the nail correction device according to one embodiment of the present invention, since a nail may be easily correct at home and a corrected state thereof may be continuously maintained, inconvenience of recorrection is prevented.

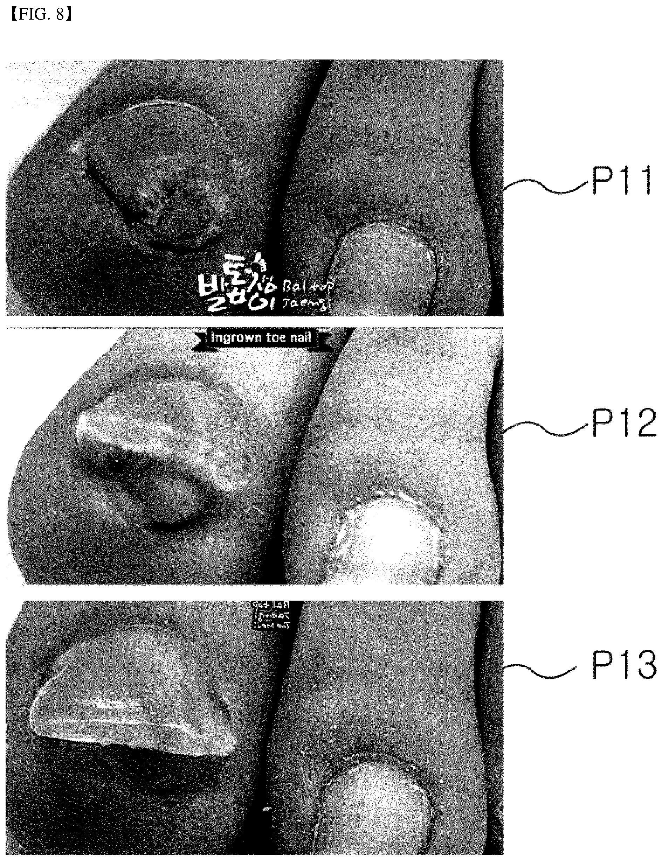

Hereinafter, an example of correction using the nail correction device according to one embodiment of the present invention will be described with reference to FIGS. 8 to 11.

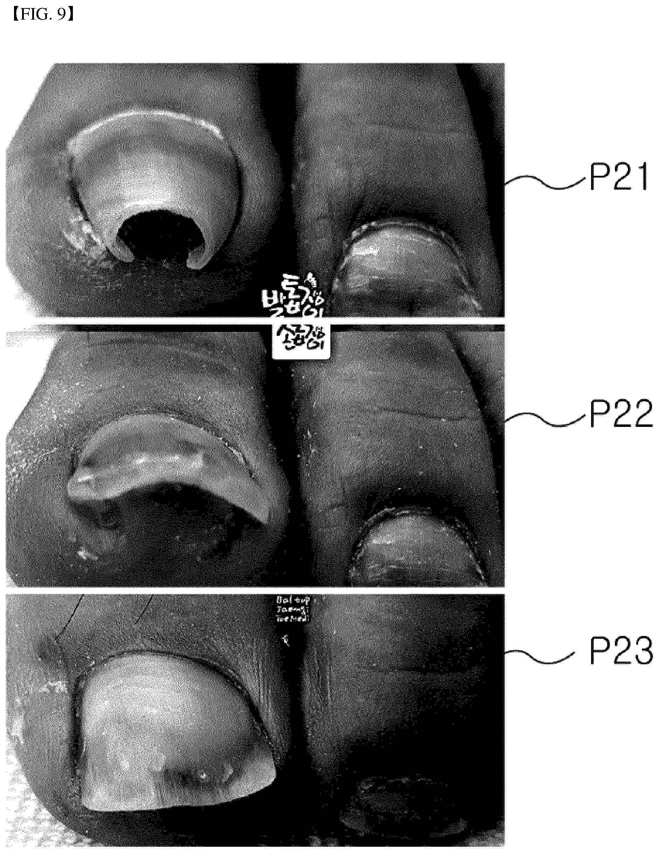

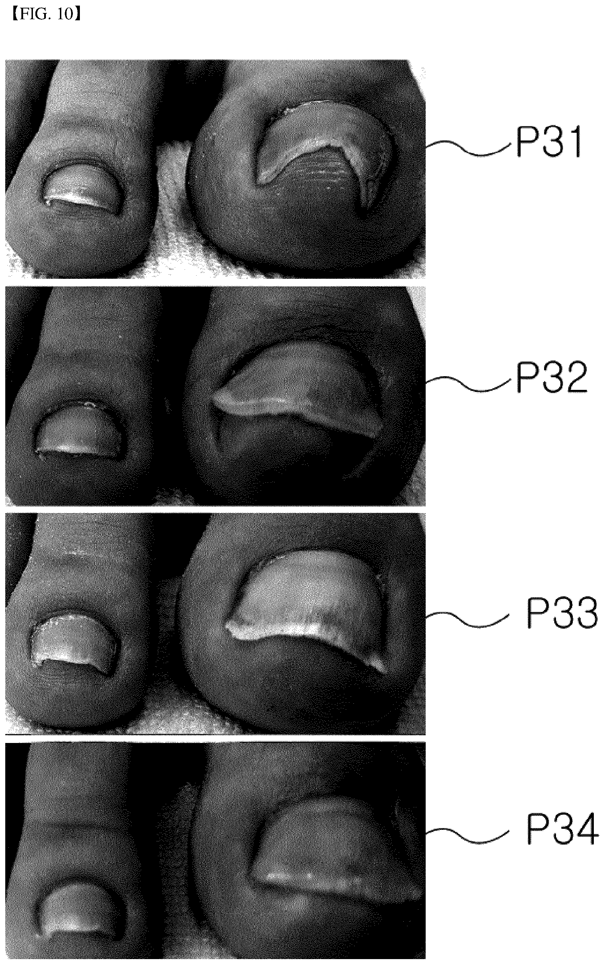

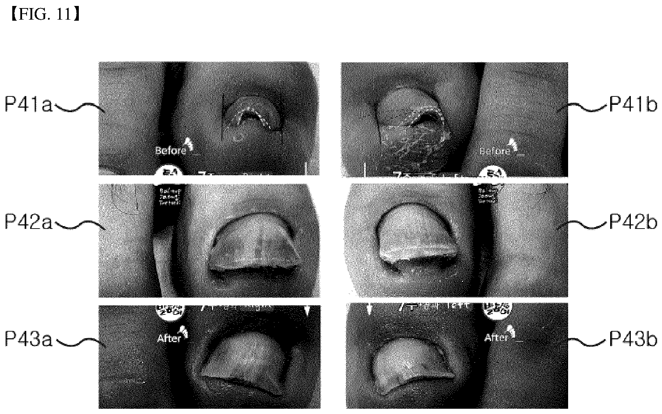

FIG. 8 is a photo illustrating a first example in which correction is performed using the nail correction device according to one embodiment of the present invention. FIG. 9 is a photo illustrating a second example in which correction is performed using the nail correction device according to one embodiment of the present invention. FIG. 10 is a photo illustrating a third example in which correction is performed using the nail correction device according to one embodiment of the present invention. FIG. 11 is a photo illustrating a fourth example in which correction is performed using the nail correction device according to one embodiment of the present invention.

The first example is an example in which a toenail of a big toe of a left foot is corrected. In FIG. 8, P11 is a state before correction, P12 is a corrected state, and P13 is a state seven weeks after correction. In P11 of FIG. 8, it may be seen that a left end of the toenail is particularly ingrown. In P13, it may be seen that as a wound caused by the ingrown toenail heals after seven weeks after correction and is filled up with the flesh, and the toenail does not further grow into the flesh.

The second example is an example in which a toenail of a big toe of a left foot is corrected. In FIG. 9, P21 is a state before correction, P22 is a corrected state, and P23 is a state seven weeks after correction. P21 is a state in which both ends of the toenail are growing inward. In P22, the toenail is cleaned up by lifting the both ends of the toenail, which grows into the flesh, using the nail correction device of the present invention. Seven weeks thereafter, as shown in P23, it may be seen that the toenail is restored to a normal toenail shape and does not further grow into the flesh.

The third example is an example in which a toenail of a big toe of a left foot is corrected. In FIG. 10, P31 is a state before correction, P32 is a corrected state, P33 is a state seven weeks after correction, and P34 is a state after trimming the toenail of P33. P31 is a state in which a left end of the toenail grows further inward than a right end. In P32, both ends of the toenail, which grows into the flesh, are lifted and corrected using the nail correction device of the present invention. Seven weeks thereafter, as shown in P33, the flesh, into which the toenail grows, is completely filled to become a normal toe state. In P34, it may be seen that after being trimmed, the toenail becomes a completely normal toenail shape.

The fourth example is an example in which toenails of big toes of a right foot and a left foot are corrected. In FIG. 11, P41a, P42a, and P43a are a state before correction, a corrected state, and a state seven weeks after correction of the toenail of the right foot. P41b, P42b, and P43b are a state before correction, a corrected state, and a state seven weeks after correction of the toenail of the left foot. As shown in FIG. 11, in the fourth example, both ends are growing inward (P41a and P41b), and particularly, a right end of the toenail of the left foot is distorted and grows inward (P41b). In P42a and P42b, both ends of the toenail, which grow into the flesh, are lifted and corrected using the nail correction device of the present invention. Seven weeks thereafter, as shown in P43a and P43b, it may be seen that the flesh, into which the toenail grows, is completely filled to become a normal toe state and the toenail also becomes a normal shape.

As described above, when correction is performed using the nail correction device according to one embodiment, since a wound caused by an ingrown toenail heals and is filled with the flesh to become a normal toe state and the toenail does not grow into the flesh, a case, in which a nail grows again into the flesh after correction and needs to be recorrected, does not occur.

Although the exemplary embodiment of the present invention has been described above, it may be understood by one of ordinary skill in the art that a variety of modifications and changes may be made without departing from the concept and scope of the present invention disclosed within the range of the following claims.

## Claims

1. A nail correction device comprising:
a first handle portion and a second handle portion which each have one end at which an insertion portion into which a user's finger is inserted is formed and which are cross-coupled with each other using a hinge member to be pivotably connected to each other;
a body portion formed to extend from the other end of the first handle portion and disposed in a lateral direction of a nail;
holding portions having top ends connected to the body portion and bottom ends bent upward to penetrate under and hold both ends of the nail; and
a pressurizing portion formed to extend from the other end of the second handle portion, to come into contact with a top surface of the nail, and to pressurize the top surface of the nail.

2. The nail correction device of claim 1, wherein the body portion comprises a frame formed in the lateral direction of the nail, first and second arms connected to both ends of the frame, and a connection member configured to connect the frame to the first and second arms such that the first or second arm moves in the lateral direction according to a width of the nail.

3. The nail correction device of claim 2, wherein the body portion further comprises a fixing member configured to fix positions of the first and second arms with an adjusted lateral interval.

4. The nail correction device of claim 3, wherein the fixing member is formed as a bolt and the first or second arm is formed as a nut.

5. The nail correction device of claim 1, wherein the holding portions have a width which gradually decreases in a downward direction.

6. The nail correction device of claim 1, wherein the holding portions each comprise a tapered portion inclined inward in the lateral direction between the top end and the bottom end.

7. The nail correction device of claim 1, wherein the pressurizing portion is pivotably connected to the other end of the second handle portion by a hinge.

8. The nail correction device of claim 1, wherein the first handle portion and the second handle portion are formed to be perpendicular or parallel to the body portion.

9. A method of correcting a nail, comprising:
preparing the nail correction device according to any one of claims 1 to 8;
applying a solid formulation to a top surface of a nail;
disposing the nail correction device on the nail and mounting the holding portions on both ends of the nail;
lifting and correcting the both ends of the nail under which the holding portions penetrate while the first handle portion and the second handle portion are gripped and the pressurizing portion comes into contact with the top surface of the nail;
maintaining a corrected state of the both ends of the nail for a certain time; and
detaching the nail correction device from the nail.
